# EUROPEAN PATENT APPLICATION

(11) **EP 1 364 650 A1**
(43) Date of publication of application: **26.11.2003**
(21) Application number: 02716445.8
(22) Date of filing: 29.01.2002
(51) Int. Cl.: A61K 31/445, A61K 31/155, A61K 31/40, A61K 45/00, A61K 9/16, A61K 47/26, A61K 47/38, A61P 7/02

(54) **MEDICINAL COMPOSITION**

(30) Priority: 30.01.2001 JP 2001021473
(71) Applicant: TEIJIN LIMITED, Osaka-shi Osaka 541-0054 (JP)
(72) Inventor: FUJINAGA, Kentaro, c/o Teijin Limited, Hino-shi, Tokyo191-0065 (JP); DOHI, Masahiko, Teijin Limited, Iwakuni Factory, Iwakuni-shi, Yamaguchi 740-0014 (JP); NARASAKI, Masahiko, Teijin Limited, Hino-shi, Tokyo 191-0065 (JP); AKASOFU, Wataru, Teijin Limited, Hino-shi, Tokyo 191-0065 (JP); UEJIMA, Yasuhide, Teijin Limited, Hino-shi, Tokyo 191-0065 (JP)
(74) Representative: Hallybone, Huw George
(86) International application number: JP0200645
(87) International publication number: WO02060448

(57) **Abstract**

A pharmaceutical composition comprising a biphenylamidine derivative, particles for nucleus and a binder. Specifically, a pharmaceutical composition obtained by coating the particles for nucleus with the biphenylamidine derivative and the binder.

## Description

### Technical Field

The present invention relates to a pharmaceutical composition of a biphenylamidine derivative for oral administration, and particularly, to a pharmaceutical composition obtained by coating particles for nucleus with the biphenylamidine derivative together with a binder.

### Background Art

A biphenylamidine derivative has an FXa-inhibiting activity, and it is expected as an antithrombotic drug. Antithrombotic drugs having the FXa-inhibiting activity as their mechanism of action and available from the present market are only injections, and there is no oral preparation of such drugs. However, injections can not be an optimum dosage form when patient's compliance is considered. This is because injections have demerits, for example, the patient is suffered from pain and has to go to hospital when the drug is administered. Accordingly, it is desired to develop an oral preparation of an antithrombotic drug with which the antithrombotic drug can be administered in an easier manner.

It is the most important problem in the development of oral preparations of a drug that the drug has a sufficient solubility, and it is sufficiently absorbed in the digestive tract. Although biphenylamidine derivatives have a high solubility in water, not less than 30 mg/mL, the absorptions of the derivatives in rats are vary low and of several %. Accordingly, it is a problem in the development of an oral preparation of a biphenylamidine derivative to make it easily absorbable in the digestive tract.

The inventors of the present invention have zealously studied for solving the above-mentioned problem, and they invented an oral preparation which has an extremely high dissolution rate, releases the biphenylamidine derivative quickly in the digestive tract and enables the excellent absorption of the derivative with the increase of the local density of the drug.

The purpose of the present invention is to provide an oral preparation wherein a biphenylamidine derivative is excellently absorbed.

### Disclosure of the Invention

The present invention provides a pharmaceutical composition comprising a biphenylamidine derivative, particles for nucleus and a binder. Specifically, the present invention provides a pharmaceutical composition wherein the particles for nucleus are coated with the biphenylamidine derivative and the binder.

### Best Mode for Carrying Out the Invention

A biphenylamidine derivative of the present invention (hereafter, this is sometimes referred to as "the drug of the present invention" or "the active component") is a preventive or curing agent which has an FXa-inhibiting activity, and whose clinical application on thromboembolic diseases as an antithrombotic agent is expected. Examples of the biphenylamidine derivatives include compounds described in the specification of WO 99/26918. Among them, 3-(3-amidinophenyl)-5-acetoimidoyl-4-piperidinyl)methylaminomethyl)benzoic acid or its hydrochloride is especially preferable.

The content of the biphenylamidine derivative in a composition of the present invention is determined by the desired dose of a final product, but it is preferably in the range of about 5 wt.% to about 50 wt.%. When it exceeds 50 wt.%, occasionally the biphenylamidine derivative is not adhered sufficiently on the particles for nucleus due to the shortage of a binder, and the coating efficiency is lowered.

The particles for nucleus of the present invention have particle sizes preferably in the range of 100 µm to 800 µm so that the active component and the binder are applied on their surfaces. As for the shape, sphere or a shape close to sphere is preferable because it has a high coating efficiency. Concretely, spherical sucrose particles such as NONPAREIL (registered trademark) and spherical crystalline cellulose particles such as CELPHERE (registered trademark) are preferable as the particles for nucleus. Especially, CELPHERE (registered trademark) is preferred in that CELPHERE is suitable for an aqueous coating process since it is physically stable so that it hardly has cracks and chips during coating, and it is not soluble in water.

The binder of the present invention is required to have such properties that it does not interact with the biphenylamidine derivative and allows the derivative to be dissoluted quickly in an organism, especially in the digestive tract. From such a view point, hydroxypropylcellulose (hereafter, this is sometimes referred to as "HPC"), one of cellulose derivatives, can be cited as a preferable example.

There are several grades having different molecular weights in the HPC. The HPC of the present invention is selected considering following factors: correlation between the viscosity of a 2 wt.% HPC solution and the dissolution effect of the drug of the present invention; the easiness of spraying during coating; and the lowness of adhesivity and coagulation among granules during the coating. A HPC whose 2 wt.% aqueous solution has a viscosity not larger than 100 cps, especially of 3 cps to 10 cps, is preferable as the HPC. A concrete example of the preferable HPC is HPC-L (registered trademark), HPC-SL (registered trademark) or the like.

The content of the HPC in a pharmaceutical composition of the present invention is determined depending on the amount of the active component, but it is preferably in the range of about 2 wt.% to about 20 wt.%. When it is not larger than 2 wt.%, there is the possibility of insufficient adhesion of the active component on the particles for nucleus due to the lack of the binder during manufacturing to lower coating efficiency. When it exceeds 20 wt.%, there is the possibility of delayed dissolution of the active component from the composition due to excess of the binder to lower the absorbability in oral administration.

In order to coat the particles for nucleus with the drug of the present invention and the binder, it is preferable that a suspension prepared by dissolving or dispersing the drug and the binder in a solvent is applied by spray coating.

As the solvent, a medically and pharmaceutically permissible water such as purified water, sterilized purified water or distilled water for injection is preferable. Further, a pH-adjusting agent may be added to the solvent to make the coating liquid acidic. Examples of the pH-adjusting agent include hydrochloric acid, sulfuric acid, acetic acid, phosphoric acid and citric acid.

Concretely, the above-mentioned coating method is carried out as follows. At first the binder is dissolved in the solvent, a powder of the drug of the present invention is added to the obtained solution and they are well stirred to make the agent suspend and disperse in the solution. Subsequently, the obtained suspension (hereafter, this is sometimes referred to as "coating liquid" ) is applied on the surfaces of the particles for nucleus by spray coating. In the spray coating process, a fluid bed granulator, a tumbling fluidized bed granulator, a centrifugal tumbling granulator or the like, which is widely used as a granule coating machine, is used. Especially, the tumbling fluidized bed granulator is preferred because it causes little adhesion among granules and has excellent coating efficiency of the active component during manufacturing.

Further, the concentration of the binder in the coating liquid is preferably in the range of 1 wt.% to 5 wt.% because of easiness of spray coating. When the concentration is less than 1 wt.%, it is too low, and this brings insufficient adhesion of the active component on the particles for nucleus during the coating and lowers coating efficiency unpreferably. When the concentration exceeds 5 wt.%, the viscosity of the coating liquid is too high, and sometimes this makes homogeneous spraying impossible.

Furthermore, the concentration of the active component in the coating liquid is also an important factor for achieving excellent absorption, which is one of the purposes of the present invention. The concentration of the active component is preferably in the range of 3 wt.% to 20 wt.%. When the concentration in the coating liquid is less than 3 wt.%, the dissolution of the active component is retarded, and this lowers the absorption of the agent. Further, the lower concentration lengthens the manufacturing time, and causes the lowering of the coating efficiency. The case of exceeding 20 wt.% is not preferable because the active component does not adhere sufficiently on the particles for nucleus in coating, and coating efficiency is lowered.

The pharmaceutically advantageous point of a composition of the present invention is a high dissolution rate of the active component. The composition is a rapid releasing granular preparation in which almost all part of the active component is released in about 5 minutes in a dissolution test (the paddle method according to the Japanese Pharmacopoeia). The orally administered composition releases the active component quickly at the upper part of the digestive tract, the concentration of the active component at the upper part of the digestive tract is increased, and this brings an effect in which the absorption of the active component from the digestive tract. is accelerated.

To a composition of the present invention, a known lubricant, diluent, coloring agent, preservative, antiseptic, flavoring agent or the like may be added if necessary in order to improve its physical property, appearance, smell or the like as a pharmaceutical preparation. For example, talc, stearic acid, its salt, a wax or the like is added as the lubricant; starch, lactose or the like as the diluent; Red No.2 or the like as the coloring agent; ascorbic acid or the like as the preservative; a p-hydroxybenzoic ester or the like as the antiseptic and menthol or the like as the flavoring agent. Especially, it is effective to add a lubricant to suppress static electricity which is generated due to the contact among the members of the composition, in the case of large production of a composition of the present invention.

The particle sizes of a composition of the present invention depend on the particle sizes of the used particles for nucleus, and the amounts of the active component and the binder which are applied on the particles for nucleus, but they are preferably in the range of 100 µm to 1,200 µm.

Further, the composition of the present invention may be filled in capsules in order to be administered effectively. The raw material of the capsules is preferably gelatin, hydroxypropylmethylcellulose (HPMC) or the like. The size of the capsules can be arbitrarily selected from No. 00, No. 0, No. 1, No. 2, No. 3 and so on, as far as they are medically permissible.

### Examples

The present invention will be explained hereafter with examples, while the present invention is not limited by the examples.

### Example 1

Into a tumbling fluidized bed granulator (New Marumeraizer NQ-125 manufactured by Fuji Paudal Co. Ltd.), 500 g of CELPHERE (manufactured by Asahi Chemical Industry Co., Ltd.) was charged, and CELPHERE was fluidized with an air flow of 0.7 m³/min at 45 °C. Subsequently, a coating liquid was prepared by homogeneously suspending 3-(3-amidinophenyl)-5-({[(1-acetoimidoyl-4-piperidinyl)methyl)amino}methyl)benzoic acid (hereafter, this is sometimes referred to as "Drug 1") homogeneously in an aqueous solution of hydroxypropylcellulose (HPC-L manufactured by Nippon Soda Co., Ltd.) to obtain a coating liquid. The concentrations of the HPC-L and the Drug 1 in the coating liquid were 3 wt.% and 7.5 wt.%, respectively. The coating liquid was applied on the fluidized NONPAREIL or CELPHERE by spray coating at 6.0 g/min, and the coated product was dried at 60°C to obtain granules containing about 15 wt.% of the Drug 1.

### Example 2

Three kinds of granules were prepared by using hydroxypropylcellulose (HPC-L manufactured by Nippon Soda Co., Ltd.), hydroxypropylmethylcellulose (TC-5 (registered trademark) manufactured by Shin-Etsu Chemical Industry Co., Ltd.) or polyvinylpyrrolidone (Kollidon 30 (registered trademark) manufactured by BASF Japan Ltd.), respectively as a binder. The concentrations of the binder and the suspended Drug 1 were 3 wt.% and 7.5 wt:%, respectively. Other manufacturing conditions were same as those shown in Example 1. About 150 mg (this amount of the granules corresponds 23 mg of the Drug 1) of each kind of the obtained granules was filled in a gelatin capsule of size number of 00 (manufactured by Shionogi Qualicaps Co. Ltd.).

0.12 mg/mL/kg of loperamide hydrochloride suspension was administered orally to a beagle dog under starvation together with 15 mL of ion exchanged water; and 30 minutes later, a piece of the obtained capsules was administered, and immediately 20 mL of water was given. Blood samples were taken serially after administration, and the change of the plasma concentrations of the Drug 1 was determined.

AUC (Area Under the Curve, i.e. an area under a curve of blood concentration of the drug vs, time), which is a pharmacodynamic parameter obtainable from the result, was calculated for each kind of the granules, and they are shown in Table 1.

**Table 1**

| | binder | AUC (ng•hr/mL) |
|---|---|---|
| 1 | hydroxypropylcellulose (HPC-L) | 2230 |
| 2 | hydroxypropylmethylcellulose (TC-5) | 1718 |
| 3 | polyvinylpyrrolidone (Kollidon 30) | 1572 |

### Example 3

Three kinds of granules were prepared by using each of the grades (HPC-M, HPC-L and HPC-SL) of hydroxypropylcellulose as the binder of a composition of the present invention. The concentrations of the binder and the suspended Drug 1 were 3 wt.% and 7.5 wt.%, respectively. Other manufacturing conditions were same as those shown in Example 1.

The agglomerate ratios (the weight of the granules remaining on a 20-mesh sieve / the whole weight of the granules) of the finished granules obtained by spray coating with each of HPC-M, -L and -SL as the binder were calculated, and agglomerating properties among granules during manufacturing were evaluated. The results are shown in Table 2.

**Table 2**

| binder | agglomerate ratio (%) |
|---|---|
| HPC-M | 8 |
| HPC-L | ≦ 1 |
| HPC-SL | ≦ 1 |

### Example 4

The effect of the concentration of a hydroxypropylcellulose (HPC-L) in a coating liquid on the coating efficiency of Drug 1 in the manufacturing of a composition of the present invention was studied.

Seven kinds of granules having 7.5 wt.% of Drug 1 were prepared, wherein the concentration of the HPC-L of each granule was 0.5 wt.%, 0.75 wt.%, 1 wt.%, 3 wt.%, 5 wt.%, 7.5 wt.% and 10 wt.%, respectively. The remaining conditions were same as those of Example 1. The results of the coating efficiencies of Drug 1 in the obtained granules were calculated, and they are shown in Table 3. The concentration of the HPC-L in the coating liquid ranging from 1 wt.% to 5 wt.% gives a good coating efficiency of Drug 1, and the concentrations of 0.5 wt.% and 0.75 wt.% give low coating efficiencies of Drug 1. Further, when the concentration is 7.5 wt.% or 10 wt.%, the homogeneous spraying of the coating liquid is difficult, and the adhesion and agglomeration of granules become large to result in a low coating efficiency of Drug 1 in the granules.

**Table 3**

| concentration (wt.%) of HPC-L in coating liquid | coating efficiency (%) |
|---|---|
| 0.5 | 44 |
| 0.75 | 52 |
| 1 | 78 |
| 3 | 89 |
| 5 | 84 |
| 7.5 | 61 |
| 10 | 39 |

### Example 5

The influence of the difference in the concentration of Drug 1 in a coating liquid affecting on the coating efficiency of Drug 1 was studied in the manufacturing of a composition of the present invention. Seven kinds of granules having 3 wt.% of the binder were prepared, wherein the concentration of Drug 1 of each granule was 0.5 wt.%, 1 wt.%, 3 wt.%, 5 wt.%, 10 wt.%, 20wt.% and 30 wt.%, respectively. The remaining conditions were same as those in Example 1. The coating efficiencies of Drug 1 in the finished granules were calculated, and they are shown in Table 4. The concentrations of Drug 1 in the coating liquid ranging from 3 wt.% to 10 wt.% give relatively good coating efficiencies, but the concentrations of 0.5 wt.%, 1 wt.%, 20 wt.% and 30 wt.% give low coating efficiencies.

**Table 4**

| concentration (wt.%) of Drug 1 in coating liquid | coating efficiency (%) |
|---|---|
| 0.5 | 52 |
| 1 | 63 |
| 3 | 84 |
| 5 | 92 |
| 10 | 92 |
| 20 | 52 |
| 30 | 35 |

### Reference Example 1

Drug 1-containing uncoated tablets were prepared as follows. After 12.50 g of Drug 1, 114.25 g of lactose (DILACTOSE R) and 2.6 g of Croscarmellose sodium (AcDiSol manufactured by Asahi Chemical Industry Co., Ltd.) were mixed for 2 min with a high-speed mixer granulator (FDG-C-5 manufactured by FUKAE POWTEC), 0.65 g of magnesium stearate was added, and the mixture was mixed for 10 sec. The mixed powder was pressed with a single punch tableting machine (KORSCH) to obtain tablets having the content of the active component of 11.5 mg/tablet, an average weight of 131.3 mg and a diameter of 7 mm.

### Reference Example 2

Enteric coated tablets containing Drug 1 were prepared as follows. After 28.8 g of Drug 1, 263.7 g of lactose and 2.6 g of Croscarmellose sodium were mixed for 2 min with a high-speed mixer granulator, magnesium stearate was added, and the mixture was mixed for 10 sec. The mixed powder was pressed with a single punch tableting machine to obtain tablets having a diameter of 7 mm. A coating liquid consisting of 554.1 g of purified water, 30.0 g of hydroxypropylmethylcellulose acetate succinate (AQOAT AS-LF manufactured by Shin-Etsu Chemical), 6.0 g of triethyl citrate (Citroflex 2, SC-60), 9.0 g of talc (manufactured by Matumura Sangyou) and 0.9 g of sodium lauryl sulfate (manufactured by Nikkou Chemical) was applied on the tablets by spray coating using a coating machine (HCT-MINI manufactured by Freund Sangyo) to obtain enteric coated tablets. The uncoated tablet in an amount of 250 g was charged into the coating process, wherein a heater was set at 60°C. The content of Drug 1 in the obtained enteric coated tablets was 11.5 mg/tablet.

### Example 6

Absorption properties were compared among the Drug 1-containing granules prepared in Example 1, the Drug 1-containing uncoated tablets prepared in Reference Example 1 and the Drug 1-containing enteric coated tablets prepared in Reference Example 2. The evaluation was carried out by using beagle dogs (n=3). The doses of the Drug 1-containing uncoated tablets and the Drug 1-containing enteric coated tablets were each two tablets (the content of Drug 1 was 23 mg per 2 tablets). As for the Drug 1-containing granules, about 150 mg of the granules, i.e. an amount equivalent to 23 mg of Drug 1, was filled in a gelatin capsule of a size number of 00, and the dose was one capsule. A loperamide hydrochloride suspension of 0.12 mg/mL/kg was administered orally to a beagle dog under starvation together with 15 mL of ion exchanged water, and 30 minutes later, the Drug 1-containing uncoated tablet, enteric coated tablet or granules was administered each at the above-mentioned dose, and immediately 20 mL of ion-exchanged water was given. Blood samples were taken serially after administration, and the change of the plasma concentrations of Drug 1 was determined. Cmax (the maximum blood concentration), Tmax (time when the maximum blood concentration is obtained) and AUC, which are pharmacodynamic parameters obtainable from the results, were calculated, and they are shown in Table 5. The composition of the present invention prepared in Example 1 is the highest in both the Cmax and AUC, and it has been confirmed that the composition of the present invention has the best absorption of Drug 1 among 3 kinds of pharmaceutical preparations.

**Table 5**

| dosage form | Cmax (ng/mL) | Tmax (hr) | AUC (ng•hr/mL) |
|---|---|---|---|
| Example 1 | 873 | 1 | 2230 |
| Reference Example 1 | 318 | 1 | 1234 |
| Reference Example 2 | 243 | 3 | 1172 |

### Example 7

Drug 1-containing granules prepared in Example 1 and Drug 1-containing uncoated tablets prepared in Reference Example 1 were examined on their dissolutions by the paddle method according to the Japanese Pharmacopoeia. A piece of the uncoated tablet or 75 mg of the granules was charged each into 900 mL of the first solution (pH 1.2) and 900 mL of the second solution (pH 6.8) according to the Japanese Pharmacopoeia, and their dissolutions of Drug 1 were determined under conditions of 37°C and 50 rpm. The Drug 1-containing uncoated tablet exhibited the dissolution of Drug 1 of about 70% at 5 min and about 90% at 10 min in the first solution and the second solution according to the Japanese Pharmacopoeia, respectively. However, the Drug 1-containing granules exhibited about 99% dissolution of the Drug 1 at 5 min in both the solutions. From the results, it has been confirmed that the Drug 1-containing granules are rapidly releasing granules whose dissolution rate is higher than that of the uncoated tablet.

## Claims

1. A pharmaceutical composition comprising a biphenylamidine derivative, particles for nucleus and a binder.

2. The pharmaceutical composition according to Claim 1, wherein the particles for nucleus are coated with the biphenylamidine derivative and the binder.

3. The pharmaceutical composition according to Claim 1 or 2, wherein the particles for nucleus are sucrose or crystalline cellulose.

4. The pharmaceutical composition according to Claim 3, wherein the sucrose or the crystalline cellulose for nucleus is a spherical sucrose or a spherical crystalline cellulose, respectively, and has particle sizes in the range of 100 µm to 800 µm.

5. The pharmaceutical composition according to Claim 4, wherein the spherical sucrose or the spherical crystalline cellulose has particle sizes in the range of 300 µm to 500 µm.

6. The pharmaceutical composition according to Claim 4 or 5, wherein the spherical sucrose is NONPAREIL (registered trademark) and the spherical crystalline cellulose is CELPHERE (registered trademark).

7. The pharmaceutical composition according to Claim 1 or 2, wherein the binder is hydroxypropylcellulose.

8. The pharmaceutical composition according to Claim 7, wherein a 2 wt.% aqueous solution of the hydroxypropylcellulose has a viscosity of 3 cps to 10 cps.

9. The pharmaceutical composition according to Claim 7, wherein the content of the hydroxypropylcellulose is in the range of 2 wt.% to 20 wt.%.

10. The pharmaceutical composition according to Claim 1 or 2, wherein the content of the biphenylamidine derivative is in the range of 5 wt.% to 50 wt.%.

11. A pharmaceutical composition manufactured by a process comprising the steps of;
preparing a suspension by suspending a biphenylamidine derivative in an aqueous solution of a binder, and subsequently
spray coating sucrose, or crystalline cellulose with the suspension by using a tumbling fluidized bed granulator.

12. The pharmaceutical composition according to Claim 11, wherein the concentration of the aqueous solution of the binder is in the rang of 1 wt.% to 5 wt.%.

13. The pharmaceutical composition according to Claim 11, wherein the sucrose or the crystalline cellulose is a spherical sucrose or a spherical crystalline cellulose, and it has particle sizes in the range of 100 µm to 800 µm.

14. The pharmaceutical composition according to Claim 13, wherein the spherical sucrose or the spherical crystalline cellulose has particle sizes in the range of 300 µm to 500 µm.

15. The pharmaceutical composition according to Claim 13 or 14, wherein the spherical sucrose is NONPAREIL (registered trademark) and the spherical crystalline cellulose is CELPHERE (registered trademark).

16. The pharmaceutical composition according to Claim 11, wherein the binder is hydroxypropylcellulose.

17. The pharmaceutical composition according to Claim 16, wherein a 2 wt.% aqueous solution of the hydroxypropylcellulose has a viscosity of 3 cps to 10 cps.

18. The pharmaceutical composition according to Claim 16 or 17, wherein the content of the hydroxypropylcellulose is in the range of 2 wt.% to 20 wt.%.

19. The pharmaceutical composition according to Claim 11, wherein the concentration of the biphenylamidine derivative in the suspension is in the range of 3 wt.% to 10 wt.%.

20. The pharmaceutical composition according to Claim 11, wherein the content of the biphenylamidine derivative is in the range of 5 wt.% to 50 wt.%.

21. The pharmaceutical composition according to Claim 1 or 11, wherein the biphenylamidine derivative is quickly released at the upper part of the digestive tract after administration, and the absorption of the compound into the body is accelerated due to the increase of the concentration of the compound at the upper part of the digestive tract.

22. The pharmaceutical composition according to Claim 1 or 11, wherein the dissolution rate of the biphenylamidine derivative is 95% to 100% within 5 min in both the JP1 solution and the JP2 solution in a dissolution test by the paddle method according to the Japanese Pharmacopoeia.

23. The pharmaceutical composition according to Claim 1 or 11 additionally containing talc.

24. The pharmaceutical composition according to Claim 1 or 11, wherein the particle sizes of the pharmaceutical composition are in the range of 100 µm to 1200 µm.

25. The pharmaceutical composition according to Claim 1 or 11, wherein the biphenylamidine derivative is 3-(3-amidinophenyl) -5 -((1-acetoimidoyl-4-piperidinyl)methylaminomethyl)benzoic acid or its pharmaceutically permissible salt.

26. An encapsulated pharmaceutical composition comprising the pharmaceutical composition according to any one of the Claims 1 to 25.
